# EUROPEAN PATENT SPECIFICATION

(11) **EP 2 026 764 B1**
(45) Date of publication and mention of the grant of the patent: **12.07.2017**
(21) Application number: 07761644.9
(22) Date of filing: 01.05.2007
(51) Int. Cl.: A61K 9/00, A61K 31/00

(54) **A SUSTAINED-RELEASE INTRAOCULAR IMPLANT COMPRISING A VASOACTIVE AGENT**
INTRAOKULARES IMPLANTAT MIT VASOAKTIVEM WIRKSTOFF MIT VERZÖGERTER FREISETZUNG
IMPLANT INTRAOCULAIRE À LIBÉRATION LENTE COMPRENANT UNE SUBSTANCE VASOACTIVE

(30) Priority: 04.05.2006 US 417420
(43) Date of publication of application: 25.02.2009
(73) Proprietor: ALLERGAN, INC., Irvine, CA 92612 (US)
(72) Inventor: DONELLO, John, E., Dana Point, CA 92629 (US); YANG, Rong, Mission Viejo, CA 92691 (US)
(74) Representative: Hoffmann Eitle
(86) International application number: PCT/US2007/067883
(87) International publication number: WO 2007/130945

(56) References cited:
- WO-A-02/074196
- WO-A-2005/110362
- WO-A-2005/110424
- WO-A-2007/011874
- US-A1- 2005 244 469
- US-B1- 6 306 426

## Description

### BACKGROUND

The present invention relates to an intraocular implant containing a vasoactive agent and use of the implant to treat an ocular condition. In particular the present invention relates to biodegradable intraocular implants containing one or more vasoactive agent and use of the implants to treat an ocular condition such as glaucoma.

A pharmaceutical composition (synonymously a composition) is a formulation which contains at least one active ingredient (for example a vasoactive agent such as a vasodilator) as well as, for example, one or more excipients, buffers, carriers, stabilizers, preservatives and/or bulking agents, and is suitable for administration to a patient to achieve a desired effect or result. The pharmaceutical compositions disclosed herein can have diagnostic, therapeutic, cosmetic and/or research utility in various species, such as for example in human patients or subjects.

An ocular condition can include a disease, aliment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball. An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves, the conjunctiva, the cornea, the conjunctiva, the anterior chamber, the iris, the posterior chamber (behind the retina but in front of the posterior wall of the lens capsule), the lens or the lens capsule and blood vessels and nerve which vascularize or innervate an anterior ocular region or site. A posterior ocular (also referred to herein synonymously as a "posterior segment") condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular (or posterior segment) region or site.

Thus, a posterior ocular condition can include a disease, ailment or condition, such as for example, macular degeneration (such as non-exudative age related macular degeneration and exudative age related macular degeneration); choroidal neovascularization; acute macular neuroretinopathy; macular edema (such as cystoid macular edema and diabetic macular edema); Behcet's disease, retinal disorders, diabetic retinopathy (including proliferative diabetic retinopathy); retinal arterial occlusive disease; central retinal vein occlusion; uveitis (including intermediate and anterior uveitis); retinal detachment; ocular trauma which affects a posterior ocular site or location; a posterior ocular condition caused by or influenced by an ocular laser treatment; posterior ocular conditions caused by or influenced by a photodynamic therapy; photocoagulation; radiation retinopathy; epiretinal membrane disorders; branch retinal vein occlusion; anterior ischemic optic neuropathy; non-retinopathy diabetic retinal dysfunction, retinitis pigmentosa and glaucoma. Glaucoma can be considered a posterior ocular condition because a therapeutic goal can be to prevent the loss of or reduce the occurrence of loss of vision due to damage to or loss of retinal cells or optic nerve cells (i.e. neuroprotection).

An anterior ocular condition can include a disease, ailment or condition, such as for example, aphakia; pseudophakia; astigmatism; blepharospasm; cataract; conjunctival diseases; conjunctivitis; corneal diseases;, corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

Macular edema is a major cause of visual loss in patients with diabetes, central retinal vein occlusion (CRVO) and branch retinal vein occlusion (BRVO). Although laser photocoagulation can reduce further vision loss in patients with diabetic macular edema (DME), vision that has already been decreased by macular edema usually does not improve by use of laser photocoagulation. Currently, there is no FDA (U.S. Food and Drug Administration) approved treatment for macular edema associated with CRVO. For macular edema associated with BRVO, grid laser photocoagulation may be an effective treatment for some patients.

Diabetic macular edema results from abnormal leakage of macromolecules, such as lipoproteins, from retinal capillaries into the extravascular space followed by an oncotic influx of water into the extravascular space. Abnormalities in the retinal pigment epithelium may also cause or contribute to diabetic macular edema. These abnormalities can allow increased fluid from the choriocapillaries to enter the retina or they may decrease the normal efflux of fluid from the retina to the choriocapillaries. The mechanism of breakdown of the blood-retina barrier at the level of the retinal capillaries and the retinal pigment epithelium may be due to changes to tight junction proteins such as occludin. Antcliff R., et al Marshall J., The pathogenesis of edema in diabetic maculopathy, Semin Ophthalmol 1999; 14:223-232.

Macular edema from venous occlusive disease can result from thrombus formation at the lamina cribrosa or at an arteriovenous crossing. These changes can result in an increase in retinal capillary permeability and accompanying retinal edema. The increase in retinal capillary permeability and subsequent retinal edema can ensue from of a breakdown of the blood retina barrier mediated in part by vascular endothelial growth factor (VEGF), a 45 kD glycoprotein, as it is known that VEGF can increase vascular permeability. VEGF may regulate vessel permeability by increasing phosphorylation of tight junction proteins such as occludin and zonula occluden. Similarly, in human non-ocular disease states such as ascites, VEGF has been characterized as a potent vascular permeability factor (VPF).

Glaucoma is a disease which results in damage to the optic nerve and loss of vision. Decreased retinal blood flow may be a factor which contributes to glaucoma, as decreased retinal circulation has been linked to glaucomatous optic nerve atrophy especially in normal-pressure and elderly patients. See eg Alm, A., Etiological and pharmacological aspects on blood flow and glaucoma*;* Kaiser, H., et al., Blood flow in retrobulbar vessels in glaucoma patients and normals*,* and; Michelson, G., et al., Retinal circulation in glaucoma*,* being respectively pages 167-173, 135-138 and 217-220 in Vascular Risk Factors and Neuroprotection in Glaucoma (1996), edited by S.M. Drance, Kugler Publications.

In glaucoma typically peripheral vision is lost first followed by total loss of vision if left untreated. The three basic types of glaucoma open angle, closed angle and congenital glaucoma. Open-angle glaucoma is a common form of glaucoma in which the optic nerve is slowly damaged with a causing gradual loss of vision. Both eyes can be affected at the same time, although one may be affected more than the other. In the less common closed-angle glaucoma (chronic and acute forms are known) the iris and the lens block the movement of fluid between the chambers of the eye, causing pressure to build up and the iris to press on the drainage system (the trabecular network) of the eye. Symptoms can include sudden blurred vision with pain and redness, usually in one eye, nausea and vomiting. Congenital glaucoma is rare.

Damage to the optic nerve can be due to increased pressure in the eye (i.e. elevated intraocular pressure). Elevated intraocular pressure (IOP) (ocular hypertension) can result from excess aqueous humor accumulating because the eye either produces too much or drains too little aqueous humor. Notably glaucoma can develop without concurrent ocular hypertension, in which circumstances decreased blood flow to the optic nerve may be a cause of the damage which results in vision loss.

Glaucoma can develop after an eye injury, eye surgery, growth of an eye tumor, or as a complication of a medical condition such as diabetes. Certain medications such as corticosteroids can cause glaucoma when they are used to treat eye inflammation or other diseases. Glaucoma that develops as a result of another condition is called secondary glaucoma.

Treatment for glaucoma focuses on preserving eyesight by slowing the damage to the optic nerve. Most treatment aims to prevent further damage to the optic nerve by lowering IOP. Glaucoma is usually treated with medications such as eyedrops. Laser treatment or surgery can also be practised to treat glaucoma. Topical beta blockers used to treat glaucoma include timolol (Timoptic), betaxolol (Betoptic), levobunolol (Betagan), carteolol (Ocupress), and metipranolol (OptiPranolol). Topical prostaglandins are also proving to be very beneficial alternatives if beta blockers fail and can include latanoprost (Xalatan) and unoprostone (Rescula). Topical carbonic anhydrase inhibitors (CAIs) are less effective than standard beta blockers but can also be used. Topical forms are dorzolamide (Trusopt) and brinzolamide (Azopt). Oral CAIs are available and more effective, but they have severe side effects and are rarely used for long term treatment. Alpha2-adrenergics, also called selective alpha adrenergics are effective but may not be well tolerated. They include brimonidine (Alphagan, Allergan). Nonselective alpha adrenergics include older drugs, such as epinephrine. Miotics are older agents which include pilocarpine. Before the introduction of timolol there were the standard agents but have largely been replaced or are used in combinations. Older agents include miotics, oral carbonic anhydrase inhibitors, and nonselective alpha adrenergics. They can be helpful but can have severe side effects.

Unfortunately the topical drugs used to treat glaucoma have significant drawbacks, deficiencies and side effects due for example to the amount of the drug which must be applied to achieve the desired therapeutic efficacy.

It is known to make and use an intraocular implant to treat an ocular condition. See for example U.S. patents 4521210; 4853224; 4997652; 5164188; 5443505; 5766242; 5824072; 5869079; 6331313; 6726918; 6699493; 5501856; 6074661; and; 6369116, and U.S. patent applications 11/070,158; 11/292,544; 10/966,764; 11/117,879; 11/119,463; 11/116,698; 11/119,021; 11/118,519; 11/119,001; 11/118,288; 11/119,024; 10/340,237; 10/837,357; 10/837,355; 10/837,142; 10/837,356; 10/836,911; 10/837,143; 10/837,260; 10/837,379; 10/836,880, and 10/918,597.

U.S. Patent No. 6,713,081 discloses ocular implant devices made from polyvinyl alcohol and used for the delivery of a therapeutic agent to an eye in a controlled and sustained manner. The implants may be placed subconjunctivally or intravitreally in an eye. Subsequent patents deal with further forms of ocular implants comprising aye-active agents of varied nature.

What is needed therefore is an intraocular implant which can deliver a therapeutically effective amount of an active agent to retinal tissue over a sustained period such as multiweek period so as to improve retinal circulation and thereby treat glaucoma without significant systemic side effects.

### SUMMARY

The present invention met this need and provides an intraocular implant which can deliver a therapeutically effective amount of an active agent to retinal tissue over a sustained period such as multiweek period so as to improve retinal circulation and thereby treat glaucoma without significant systemic side effects according to claim 1.

### Definitions

As used herein, the words or terms set forth below have the meanings shown.

"About" means that the item, parameter or term so qualified encompasses a range of plus or minus ten percent above and below the value of the stated item, parameter or term.

"Administration", or "to administer" means the step of giving (i.e. administering) a pharmaceutical composition to a subject. The pharmaceutical compositions disclosed herein can be "locally administered", that is administered at or in the vicinity of the site at which a therapeutic result or outcome is desired. For example to treat an ocular condition (such as for example glaucoma, a macular edema, uveitis or macular degeneration) intravitreal injection or implantation of a sustained release device such as active agent containing polymeric implant can be carried out. "Sustained release" means release of an active agent (such as a vasoactive agent) over a period of at least about five to seven days and for as long as several years.

"Associated with" means mixed with, dispersed within, coupled to, covering, or surrounding.

"Biodegradable polymer" means a polymer or polymers which degrade in vivo, and wherein erosion of the polymer or polymers over time occurs concurrent with or subsequent to release of the therapeutic agent. Specifically, hydrogels such as methylcellulose which act to release drug through polymer swelling are specifically excluded from the term "biodegradable polymer". The terms "biodegradable" and "bioerodible" are equivalent and are used interchangeably herein. A biodegradable polymer may be a homopolymer, a copolymer, or a polymer comprising more than two different polymeric units.

"Entirely free (i.e. "consisting of" terminology) means that within the detection range of the instrument or process being used, the substance cannot be detected or its presence cannot be confirmed.

"Essentially free" (or "consisting essentially of") means that only trace amounts of the substance can be detected.

"Intraocular implant" means a device or element that is structured, sized, or otherwise configured to be placed in an eye. Intraocular implants are generally biocompatible with physiological conditions of an eye and do not cause unacceptable adverse side effects. Intraocular implants can be placed in an eye without disrupting vision of the eye.

"Pharmaceutical composition" means a formulation in which an active ingredient (the active agent) can be a vasoactive agent, such as a vasodilator. The word "formulation" means that there is at least one additional ingredient in the pharmaceutical composition besides the active ingredient. A pharmaceutical composition is therefore a formulation which is suitable for diagnostic or therapeutic administration (i.e. by intraocular injection or by insertion of a depot or implant) to a subject, such as a human patient.

"Substantially free" means present at a level of less than one percent by weight of the pharmaceutical composition.

"Therapeutic component" means a portion of an intraocular implant comprising one or more therapeutic agents or substances used to treat a medical condition of the eye. The therapeutic component may be a discrete region of an intraocular implant, or it may be homogenously distributed throughout the implant. The therapeutic agents of the therapeutic component are typically ophthalmically acceptable, and are provided in a form that does not cause adverse reactions when the implant is placed in an eye.

"Therapeutically effective amount" means the level or amount of agent needed to treat an ocular condition, or reduce or prevent ocular injury or damage without causing significant negative or adverse side effects to the eye or a region of the eye.

"Treat", "treating", or "treatment" means reduction or resolution or prevention of an ocular condition, ocular injury or damage, or to promote healing of injured or damaged ocular tissue.

The present invention provides new drug delivery systems, and methods of making and using such systems, for extended or sustained drug release into an eye, for example, to achieve one or more desired therapeutic effects. The drug delivery systems are in the form of implants or implant elements that may be placed in an eye. The present systems and methods advantageously provide for extended release times of one or more therapeutic agents. Thus, the patient in whose eye the implant has been placed receives a therapeutic amount of an agent for a long or extended time period without requiring additional administrations of the agent. For example, the patient has a substantially consistent level of therapeutically active agent available for consistent treatment of the eye over a relatively long period of time, for example, on the order of at least about one week, such as between about one and about six months after receiving an implant. Such extended release times facilitate obtaining successful treatment results.

Intraocular implants in accordance with the disclosure herein comprise a therapeutic component and a drug release sustaining component associated with the therapeutic component. In accordance with the present invention, the therapeutic component comprises, consists essentially of, or consists of, a therapeutic agent which is a vasoactive agent such as a vasodilator. The drug release sustaining component is associated with the therapeutic component to sustain release of an amount of the therapeutic agent into an eye in which the implant is placed. The amount of the therapeutic agent is released into the eye for a period of time greater than about one week after the implant is placed in the eye and is effective in reducing or treating an ocular condition (such as glaucoma) to improve or maintain vision of an eye of a patient.

The drug release sustaining component (which is associated with the therapeutic component) can be a polymer such as a biodegradable polymer or polymer matrix For example, the matrix may comprise a polymer selected from the group consisting of polylactides, poly (lactide-co-glycolides), polycaprolactones, and combinations thereof.

A method of making the present implants involves combining or mixing the therapeutic agent with a biodegradable polymer or polymers. The mixture may then be extruded or compressed to form a single composition. The single composition may then be processed to form individual implants suitable for placement in an eye of a patient.

The implants may be placed in an ocular region to treat a variety of ocular conditions, such as treating, preventing, or reducing at least one symptom associated with glaucoma, or ocular conditions related to excessive excitatory activity or glutamate receptor activation.

Our invention encompasses a intraocular implant for treating an ocular condition. The implant can comprise a vasoactive compound, and a carrier associated with the vasoactive compound to thereby form an intraocular implant suitable for treating an ocular condition. The vasoactive compound can be a vasodilator. The vasodilator can be a muscarinic agent (such as pilocarpine), an endothelin receptor antagonist (an ERA) (including a selective ERA such as sitaxsentan and dual ERAs which affect both endothelin A and B, such as Bosentan), a phosphodiester-5 (PDE5) inhibitor such as Vardenafil (Levitra), sildenafil and tadalafil, a vasoactive prostaglandin, an endothelin-derived relaxation factor, a vasoactive intestinal polypeptide agonist, a smooth muscle relaxant, a leukotriene inhibitor, and pharmacologically active salts, esters, prodrugs, and metabolites thereof, and combinations of any of the foregoing. The carrier of the implant can comprise a biodegradable polymer and/or a non-biodegradable polymer. It is known to make pilocarpine nanoparticles for use in topical eye drops. Kao H., et al., Characterization of pilocarpine-loaded chitosan/carbopol nanoparticles, J Pharm & Pharmaco, 58(2); 179-186 (Feb 2006).

The biodegradable polymer of the implant can be for example a polylactides (PLA), polyglycolide (PGA), poly(lactide co-glycolide (PLGA), polycaprolactone, polyanhydride, poly methyl vinyl ether maleic anhydride, polycarbonates, polyarylates, polydioxanone, polyhydroxyalkanoates, and chitosan.

The non-biodegradable polymer of the implant can be for example an ethylcellulose, ethylvinyl acetate, polystyrene, ethylene vinyl acetate copolymers, polydimethyl siloxane, polyvinyl chloride and talc.

The implant after *in vivo* placement of the implant the implant releases a therapeutically effective amount of the vasodilator over a period of up to about seven days, up to about ten days, over a period of up to about twenty days, over a period of up to about forty days, over a period of up to about sixty days, over a period of up to about eighty days, over a period of up to about one hundred days, or over a period of up to about three years.

In our implant the carrier can be associated with the vasodilator by mixing together the carrier and the implant so as to obtain a homogenous distribution of the vasodilator in the carrier.

An additional embodiment of our invention can be an intraocular implant for treating an ocular condition comprising a vasodilator compound, and a biodegradable polymer associated with the vasoactive compound, thereby forming an intraocular implant suitable for treating an ocular condition, wherein after *in vivo* placement of the implant the implant releases a therapeutically effective amount of the vasodilator compound over a period of up to about forty days.

The implant can be structured to be placed in the vitreous of the eye, the implant of can be formed as a rod, a wafer, or a particle and the implant can be made by an extrusion process.

Also within the scope of our invention is a method of making a biodegradable intravitreal implant by extruding a mixture of a vasodilator compound, and a biodegradable polymer, thereby making forming an intravitreal implant suitable for treating an ocular condition, wherein after *in vivo* placement of the implant the implant releases a therapeutically effective amount of the vasodilator compound over a period of up to about forty days.

Also within the scope of our invention is a method of improving or maintaining vision of an eye of a patient by placing a biodegradable intraocular implant in an eye of the patient, the implant comprising a therapeutic agent which is a vasodilator wherein the implant degrades at a rate effective to sustain release of an amount of the therapeutic agent from the implant effective to improve or maintain vision in the eye of the patient. according to claim 9 of present set of claims. This method can be effective to treat a retinal ocular condition, such as retinal damage, glaucoma, or a proliferative vitreoretinopathy.

The implant can be placed in the posterior of the eye, for example using a trocar or a 25-30 gauge syringe.

Also within the scope of our invention is a method for treating glaucoma by intravitreal administration of a sustained release drug delivery system comprising a biodegradable polymer and a therapeutically effective amount of a vasoactive agent associated with the polymer and a method for improving vision by intraocular placement of an intraocular implant comprising a vasoactive compound and a carrier associated with the vasoactive compound according to claim 9. Finally, our invention also encompasses a method to preventing vision loss by intraocular placement of an intraocular implant comprising a vasoactive compound and a carrier associated with the vasoactive compound.

### DESCRIPTION

Our invention is based on the discovery that a vasoactive agent can be incorporated into an implant, such as an implant made of a biodegradable polymer (such as PLGA) and used to treat a retinal disorder, such as glaucoma. As described herein, controlled and sustained administration of a therapeutic agent through the use of one or more intraocular implants may improve treatment of undesirable ocular conditions. The implants comprise a pharmaceutically acceptable polymeric composition and are formulated to release one or more pharmaceutically active agents, such as therapeutic agents selected from the group consisting of vasoactive agents, anti-angiogenesis compounds, ocular hemorrhage treatment compounds, non-steroidal anti-inflammatory agents, VEGF inhibitors, and antibiotics, over an extended period of time. The implants are effective to provide a therapeutically effective dosage of the agent or agents directly to a region of the eye to treat, prevent, and/or reduce one or more symptoms of one or more undesirable ocular conditions. Thus, with a single administration, therapeutic agents will be made available at the site where they are needed and will be maintained for an extended period of time, rather than subjecting the patient to repeated injections or, in the case of self-administered drops, ineffective treatment with only limited bursts of exposure to the active agent or agents or, in the case of systemic administration, higher systemic exposure and concomitant side effects or, in the case of non-sustained release dosages, potentially toxic transient high tissue concentrations associated with pulsed, non-sustained release dosing.

We have intraocular implants have been developed which can release drug loads over various time periods. These implants, which when inserted into an eye, such as the vitreous of an eye, provide therapeutic levels of a therapeutic agent such as a vasodilator. for extended periods of time (e.g., for about 1 week or more). The disclosed implants are effective in treating ocular conditions, such as posterior ocular conditions, such as glaucoma, and generally improving or maintaining vision in an eye.

In one embodiment of the present invention, an intraocular implant comprises a biodegradable polymer matrix. The biodegradable polymer matrix is one type of a drug release sustaining component. The biodegradable polymer matrix is effective in forming a biodegradable intraocular implant. The biodegradable intraocular implant comprises a therapeutic agent associated with the biodegradable polymer matrix. The matrix degrades at a rate effective to sustain release of an amount of the therapeutic agent for a time greater than about one week from the time in which the implant is placed in ocular region or ocular site, such as the vitreous of an eye.

The therapeutic agent can be a vasoactive compound, such as a vasodilator. The vasodilator can be for example a endothelin-derived relaxation factor, vasoactive intestinal polypeptide agonist, smooth muscle relaxant, leukotriene inhibitor, and pharmacologically active salts, esters, thereof, and combinations of any of the foregoing.

An endothelin receptor antagonist (ERA) is a drug which blocks endothelin receptors. There are two main kinds of ERAs: selective (e.g. sitaxsentan) and dual ERAs which affect both endothelin A and B (e.g. bosentan).

Endothelin is a 21-amino acid vasoconstricting peptide that plays a key part in vascular homeostasis. There are three isoforms with varying regions of expression and two key receptor types, ETA and ETB. ETA is found in smooth muscle and binding of Endothelin to ETA increases vasoconstriction and sodium retention. ETB is primarilary located on endothelial cells and activation of this receptor increases natriuresis and diuresis and NO release. Endothelin was isolated from the Israeli Borrowing Asp in a toxin called sarafotoxin. In a healthy individual a delicate balance between vasoconstriction and vasodilation is maintained by endothelin, calcitonin and other vasoconstrictors on the one hand and nitric oxide, prostacyclin and other vasodilators on the other.

Overproduction of endothelin can cause pulmonary artery hypertension. This can sometimes be treated by the use of an endothelin receptor antagonist such as bosentan or sitaxsentan. The later selectively blocks endothelin A, decreasing the vasoconstrictive actions and allowing for increased beneficial effects of endothelin B stimulation, such as nitric oxide production (although the effects of endothelin B receptors being activated depend on the type of host cells)

Sitaxsentan or sitaxsentan sodium (Thelin®) is a small molecule sodium salt that blocks the action of endothelin on the endothelin-A receptor selectively (by a factor of 6000 compared to the ER_{B}), and is undergoing FDA approval for treating pulmonary hypertension. Its main benefit compared to bosentan, a nonselective ER blocker, is expected to be less inhibition of the beneficial effects of ER_{B} stimulation, such as nitric oxide production.

The Food and Drug Administration (FDA) approved bosentan (Tracleer™; Actelion Pharmaceuticals US, Inc.) in 2001 for the treatment of Pulmonary Arterial Hypertension (PAH). Bosentan is an orally active, nonpeptide, competitive antagonist of both ET_{A} and ET_{B} (endothelin type A and B) receptors, with a slightly higher affinity for the ET_{A} receptor. Bosentan competes with Endothelin-1 (ET-1), a neurohormone that binds at the ET_{A} and ET_{B} receptors, leading to the constriction of the pulmonary arteries when it binds to ET_{A} receptors and vasodilatation when it binds to ET_{B} receptors. Concentrations of ET-1 are elevated in the plasma and lung tissue of PAH patients, therefore suggesting a pathogenic role of ET-1 in this disease.

Our implants can also include salts of the disclosed therapeutic agents. Pharmaceutically acceptable acid addition salts of the compounds of the invention are those formed from acids which form non-toxic addition salts containing pharmaceutically acceptable anions, such as the hydrochloride, hydrobromide, hydroiodide, sulfate, or bisulfate, phosphate or acid phosphate, acetate, maleate, fumarate, oxalate, lactate, tartrate, citrate, gluconate, saccharate and p-toluene sulphonate salts.

The therapeutic agent may be in a particulate or powder form and entrapped by the biodegradable polymer matrix. Usually, therapeutic agent particles in intraocular implants will have an effective average size less than about 3000 nanometers. In certain implants, the particles may have an effective average particle size about an order of magnitude smaller than 3000 nanometers. For example, the particles may have an effective average particle size of less than about 500 nanometers. In additional implants, the particles may have an effective average particle size of less than about 400 nanometers, and in still further embodiments, a size less than about 200 nanometers.

The therapeutic agent of the implant is preferably from about 10% to 90% by weight of the implant. More preferably, the therapeutic agent is from about 20% to about 80% by weight of the implant. In a preferred embodiment, the therapeutic agent comprises about 40% by weight of the implant (e.g., 30%-50%). In another embodiment, the therapeutic agent comprises about 60% by weight of the implant.

Suitable polymeric materials or compositions for use in the implant include those materials which are compatible, that is biocompatible, with the eye so as to cause no substantial interference with the functioning or physiology of the eye. Such materials preferably are at least partially and more preferably substantially completely biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1% of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, FL 1987, pp 39-90, which describes encapsulation for controlled drug delivery, can find use in the present implants.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate.

Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitation, polyesters, polyethers and combinations thereof which are biocompatible and may be biodegradable and/or bioerodible.

Some preferred characteristics of the polymers or polymeric materials for use in the present invention may include biocompatibility, compatibility with the therapeutic component, ease of use of the polymer in making the drug delivery systems of the present invention, a half-life in the physiological environment of at least about 6 hours, preferably greater than about one day, not significantly increasing the viscosity of the vitreous, and water insolubility.

The biodegradable polymeric materials which are included to form the matrix are desirably subject to enzymatic or hydrolytic instability. Water soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the implant. Different molecular weights of the same or different polymeric compositions may be included in the implant to modulate the release profile. In certain implants, the relative average molecular weight of the polymer will range from about 9 to about 64 kD, usually from about 10 to about 54 kD, and more usually from about 12 to about 45 kD.

In some implants, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the implant, where a more flexible implant is desirable for larger geometries. The % of polylactic acid in the polylactic acid polyglycolic acid (PLGA) copolymer can be 0-100%, preferably about 15-85%, more preferably about 35-65%. In some implants, a 50/50 PLGA copolymer is used.

The biodegradable polymer matrix of the intraocular implant may comprise a mixture of two or more biodegradable polymers. For example, the implant may comprise a mixture of a first biodegradable polymer and a different second biodegradable polymer. One or more of the biodegradable polymers may have terminal acid groups.

Release of a drug from an erodible polymer is the consequence of several mechanisms or combinations of mechanisms. Some of these mechanisms include desorption from the implants surface, dissolution, diffusion through porous channels of the hydrated polymer and erosion. Erosion can be bulk or surface or a combination of both. As discussed herein, the matrix of the intraocular implant may release drug at a rate effective to sustain release of an amount of the therapeutic agent for more than one week after implantation into an eye. In certain implants, therapeutic amounts of the therapeutic agent are released for more than about one month, and even for about six months or more.

The release of the therapeutic agent from the intraocular implant comprising a biodegradable polymer matrix may include an initial burst of release followed by a gradual increase in the amount of the therapeutic agent released, or the release may include an initial delay in release of the therapeutic agent followed by an increase in release. When the implant is substantially completely degraded, the percent of the therapeutic agent that has been released is about one hundred. Compared to existing implants, the implants disclosed herein do not completely release, or release about 100% of the therapeutic agent, until after about one week of being placed in an eye.

It may be desirable to provide a relatively constant rate of release of the therapeutic agent from the implant over the life of the implant. For example, it may be desirable for the therapeutic agent to be released in amounts from about 0.01 µg to about 2 µg per day for the life of the implant. However, the release rate may change to either increase or decrease depending on the formulation of the biodegradable polymer matrix. In addition, the release profile of the therapeutic agent may include one or more linear portions and/or one or more non-linear portions. Preferably, the release rate is greater than zero once the implant has begun to degrade or erode.

The implants may be monolithic, i.e. having the active agent or agents homogenously distributed through the polymeric matrix, or encapsulated, where a reservoir of active agent is encapsulated by the polymeric matrix. Due to ease of manufacture, monolithic implants are usually preferred over encapsulated forms. However, the greater control afforded by the encapsulated, reservoir-type implant may be of benefit in some circumstances, where the therapeutic level of the drug falls within a narrow window. In addition, the therapeutic component, including the therapeutic agent(s) described herein, may be distributed in a non-homogenous pattern in the matrix. For example, the implant may include a portion that has a greater concentration of the therapeutic agent relative to a second portion of the implant.

The intraocular implants disclosed herein may have a size of between about 5 µm and about 2 mm, or between about 10 µm and about 1 mm for administration with a needle, greater than 1 mm, or greater than 2 mm, such as 3 mm or up to 10 mm, for administration by surgical implantation. The vitreous chamber in humans is able to accommodate relatively large implants of varying geometries, having lengths of, for example, 1 to 10 mm. The implant may be a cylindrical pellet (e. g., rod) with dimensions of about 2 mm x 0.75 mm diameter. Or the implant may be a cylindrical pellet with a length of about 7 mm to about 10 mm, and a diameter of about 0.75 mm to about 1.5 mm.

The implants may also be at least somewhat flexible so as to facilitate both insertion of the implant in the eye, such as in the vitreous, and accommodation of the implant. The total weight of the implant is usually about 250-5000 µg, more preferably about 500-1000 µg. For example, an implant may be about 500 µg, or about 1000 µg. For non-human individuals, the dimensions and total weight of the implant(s) may be larger or smaller, depending on the type of individual. For example, humans have a vitreous volume of approximately 3.8 ml, compared with approximately 30 ml for horses, and approximately 60-100 ml for elephants. An implant sized for use in a human may be scaled up or down accordingly for other animals, for example, about 8 times larger for an implant for a horse, or about, for example, 26 times larger for an implant for an elephant.

Thus, implants can be prepared where the center may be of one material and the surface may have one or more layers of the same or a different composition, where the layers may be cross-linked, or of a different molecular weight, different density or porosity, or the like. For example, where it is desirable to quickly release an initial bolus of drug, the center may be a polylactate coated with a polylactate-polyglycolate copolymer, so as to enhance the rate of initial degradation. Alternatively, the center may be polyvinyl alcohol coated with polylactate, so that upon degradation of the polylactate exterior the center would dissolve and be rapidly washed out of the eye.

The implants may be of any geometry including fibers, sheets, films, microspheres, spheres, circular discs, plaques and the like. The upper limit for the implant size will be determined by factors such as toleration for the implant, size limitations on insertion, ease of handling, etc. Where sheets or films are employed, the sheets or films will be in the range of at least about 0.5 mm x 0.5 mm, usually about 3-10 mm x 5-10 mm with a thickness of about 0.1-1.0 mm for ease of handling. Where fibers are employed, the fiber diameter will generally be in the range of about 0.05 to 3 mm and the fiber length will generally be in the range of about 0.5-10 mm. Spheres may be in the range of about 0.5 µm to 4 mm in diameter, with comparable volumes for other shaped particles.

The size and form of the implant can also be used to control the rate of release, period of treatment, and drug concentration at the site of implantation. Larger implants will deliver a proportionately larger dose, but depending on the surface to mass ratio, may have a slower release rate. The particular size and geometry of the implant are chosen to suit the site of implantation.

The proportions of therapeutic agent, polymer, and any other modifiers may be empirically determined by formulating several implants with varying proportions. A USP approved method for dissolution or release test can be used to measure the rate of release (USP 23; NF 18 (1995) pp. 1790-1798). For example, using the infinite sink method, a weighed sample of the implant is added to a measured volume of a solution containing 0.9% NaCl in water, where the solution volume will be such that the drug concentration is after release is less than 5% of saturation. The mixture is maintained at 37°C and stirred slowly to maintain the implants in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

In addition to the therapeutic agent included in the intraocular implants disclosed hereinabove, the intraocular implants may also include one or more additional ophthalmically acceptable therapeutic agents. For example, the implant may include one or more antihistamines, one or more different antibiotics, one or more beta blockers, one or more steroids, one or more antineoplastic agents, one or more immunosuppressive agents, one or more antiviral agents, one or more antioxidant agents, and mixtures thereof.

Pharmacologic or therapeutic agents which may find use in the present systems, include, without limitation, those disclosed in U.S. Pat. Nos. 4,474,451, columns 4-6 and 4,327,725, columns 7-8.

The amount of active agent or agents employed in the implant, individually or in combination, will vary widely depending on the effective dosage required and the desired rate of release from the implant. As indicated herein, the agent will be at least about 1, more usually at least about 10 weight percent of the implant, and usually not more than about 80, more usually not more than about 40 weight percent of the implant.

In addition to the therapeutic component, the intraocular implants disclosed herein may include effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously present in amounts sufficient to maintain a pH of the system of between about 2 to about 9 and more preferably about 4 to about 8. As such the buffering agent may be as much as about 5% by weight of the total implant. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from 0.001 to about 5% by weight and preferably 0.01 to about 2% by weight.

In addition, the implants may include a solubility enhancing component provided in an amount effective to enhance the solubility of the therapeutic agent relative to substantially identical implants without the solubility enhancing component. For example, an implant may include a β-cyclodextrin, which is effective in enhancing the solubility of the therapeutic agent. The β-cyclodextrin may be provided in an amount from about 0.5% (w/w) to about 25% (w/w) of the implant. In certain implants, the β-cyclodextrin is provided in an amount from about 5% (w/w) to about 15% (w/w) of the implant.

In some situations mixtures of implants may be utilized employing the same or different pharmacological agents. In this way, a cocktail of release profiles, giving a biphasic or triphasic release with a single administration is achieved, where the pattern of release may be greatly varied.

Additionally, release modulators such as those described in U. S. Patent No. 5,869,079 may be included in the implants. The amount of release modulator employed will be dependent on the desired release profile, the activity of the modulator, and on the release profile of the therapeutic agent in the absence of modulator. Electrolytes such as sodium chloride and potassium chloride may also be included in the implant. Where the buffering agent or enhancer is hydrophilic, it may also act as a release accelerator. Hydrophilic additives act to increase the release rates through faster dissolution of the material surrounding the drug particles, which increases the surface area of the drug exposed, thereby increasing the rate of drug bioerosion. Similarly, a hydrophobic buffering agent or enhancer dissolve more slowly, slowing the exposure of drug particles, and thereby slowing the rate of drug bioerosion.

Various techniques may be employed to produce the implants described herein. Useful techniques include, but are not necessarily limited to, solvent evaporation methods, phase separation methods, interfacial methods, molding methods, injection molding methods, extrusion methods, co-extrusion methods, carver press method, die cutting methods, heat compression, combinations thereof and the like.

Specific methods are discussed in U.S. Pat. No. 4,997,652. Extrusion methods may be used to avoid the need for solvents in manufacturing. When using extrusion methods, the polymer and drug are chosen so as to be stable at the temperatures required for manufacturing, usually at least about 85 degrees Celsius. Extrusion methods use temperatures of about 25 degrees C to about 150 degrees C, more preferably about 65 degrees C to about 130 degrees C. An implant may be produced by bringing the temperature to about 60 degrees C to about 150 degrees C for drug/polymer mixing, such as about 130 degrees C, for a time period of about 0 to 1 hour, 0 to 30 minutes, or 5-15 minutes. For example, a time period may be about 10 minutes, preferably about 0 to 5 min. The implants are then extruded at a temperature of about 60 degrees C to about 130 degrees C, such as about 75 degrees C.

In addition, the implant may be coextruded so that a coating is formed over a core region during the manufacture of the implant.

Compression methods may be used to make the implants, and typically yield implants with faster release rates than extrusion methods. Compression methods may use pressures of about 50-150 psi, more preferably about 70-80 psi, even more preferably about 76 psi, and use temperatures of about 0 degrees C to about 115 degrees C, more preferably about 25 degrees C.

The implants of the present invention may be inserted into the eye, for example the vitreous chamber of the eye, by a variety of methods, including placement by forceps or by trocar following making a 2-3 mm incision in the sclera. One example of a device that may be used to insert the implants into an eye is disclosed in U.S. Patent Publication No. 2004/0054374. The method of placement may influence the therapeutic component or drug release kinetics. For example, delivering the implant with a trocar may result in placement of the implant deeper within the vitreous than placement by forceps, which may result in the implant being closer to the edge of the vitreous. The location of the implant may influence the concentration gradients of therapeutic component or drug surrounding the element, and thus influence the release rates (e.g., an element placed closer to the edge of the vitreous may result in a slower release rate).

The present implants are configured to release an amount of the therapeutic agent effective to treat or reduce a symptom of an ocular condition, such as an ocular condition such as glaucoma. More specifically, the implants may be used in a method to tread or reduce one or more symptoms of glaucoma or proliferative vitreoretinopathy.

The implants disclosed herein may also be configured to release additional therapeutic agents, as described above. The implants set forth herein can be used to treat a variety of ocular conditions including:
Glaucoma, maculopathies/retinal degeneration: macular degeneration, including age related macular degeneration (ARMD), such as non-exudative age related macular degeneration and exudative age related macular degeneration, choroidal neovascularization, retinopathy, including diabetic retinopathy, acute and chronic macular neuroretinopathy, central serous chorioretinopathy, and macular edema, including cystoid macular edema, and diabetic macular edema. Uveitis/retinitis/choroiditis: acute multifocal placoid pigment epitheliopathy, Behcet's disease, birdshot retinochoroidopathy, infectious (syphilis, lyme, tuberculosis, toxoplasmosis), uveitis, including intermediate uveitis (pars planitis) and anterior uveitis, multifocal choroiditis, multiple evanescent white dot syndrome (MEWDS), ocular sarcoidosis, posterior scleritis, serpignous choroiditis, subretinal fibrosis, uveitis syndrome, and Vogt-Koyanagi-Harada syndrome. Vascular diseases/exudative diseases: retinal arterial occlusive disease, central retinal vein occlusion, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angitis, sickle cell retinopathy and other hemoglobinopathies, angioid streaks, familial exudative vitreoretinopathy, Eales disease. Traumatic/surgical: sympathetic ophthalmia, uveitic retinal disease, retinal detachment, trauma, laser, PDT, photocoagulation, hypoperfusion during surgery, radiation retinopathy, bone marrow transplant retinopathy. Proliferative disorders: proliferative vitreal retinopathy and epiretinal membranes, proliferative diabetic retinopathy. Infectious disorders: ocular histoplasmosis, ocular toxocariasis, presumed ocular histoplasmosis syndrome (POHS), endophthalmitis, toxoplasmosis, retinal diseases associated with HIV infection, choroidal disease associated with HIV infection, uveitic disease associated with HIV Infection, viral retinitis, acute retinal necrosis, progressive outer retinal necrosis, fungal retinal diseases, ocular syphilis, ocular tuberculosis, diffuse unilateral subacute neuroretinitis, and myiasis. Genetic disorders: retinitis pigmentosa, systemic disorders with associated retinal dystrophies, congenital stationary night blindness, cone dystrophies, Stargardt's disease and fundus flavimaculatus, Bests disease, pattern dystrophy of the retinal pigmented epithelium, X-linked retinoschisis, Sorsby's fundus dystrophy, benign concentric maculopathy, Bietti's crystalline dystrophy, pseudoxanthoma elasticum. Retinal tears/holes: retinal detachment, macular hole, giant retinal tear. Tumors: retinal disease associated with tumors, congenital hypertrophy of the RPE, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, intraocular lymphoid tumors. Miscellaneous: punctate inner choroidopathy, acute posterior multifocal placoid pigment epitheliopathy, myopic retinal degeneration, acute retinal pigment epithelitis and the like.

In one embodiment, an implant, such as the implants disclosed herein, is administered to a posterior segment of an eye of a human or animal patient, and preferably, a living human or animal. In at least one embodiment, an implant is administered without accessing the subretinal space of the eye. For example, a method of treating a patient may include placing the implant directly into the posterior chamber of the eye. In other embodiments, a method of treating a patient may comprise administering an implant to the patient by at least one of intravitreal injection, subconjuctival injection, sub-tenon injections, retrobulbar injection, and suprachoroidal injection.

In at least one embodiment, a method of treating glaucoma in a patient comprises administering one or more implants containing one or more therapeutic agents, as disclosed herein to a patient by at least one of intravitreal implantation or injection, subconjuctival injection, sub-tenon injection, retrobulbar injection, and suprachoroidal injection. A syringe apparatus including an appropriately sized needle, for example, a 22 gauge needle, a 27 gauge needle or a 30 gauge needle, can be effectively used to inject the composition with the posterior segment of an eye of a human or animal. Repeat injections are often not necessary due to the extended release of the therapeutic agent from the implants.

In another aspect of the invention, kits for treating an ocular condition of the eye are provided, comprising: a) a container comprising an extended release implant comprising a therapeutic component including a therapeutic agent as herein described, and a drug release sustaining component; and b) instructions for use. Instructions may include steps of how to handle the implants, how to insert the implants into an ocular region, and what to expect from using the implants.

### EXAMPLES

### Example 1

### Manufacture of Compressed Tablet Vasoactive Agent Implants

A vasoactive agent such as an endothelin receptor antagonists (eg Bosentan), a phosphodiester-5 inhibitor (eg Vardenafil) or pilocarpine can be used. The vasoactive agent and a poly(lactide-co-glycolide) (PLGA) can be weighed and placed in a stainless steel mixing vessel. The vessel can be sealed, placed on a Turbula mixer and mixed at a prescribed intensity such as 96 rpm for about 15 minutes. The powder blend that can result can be loaded one unit dose at a time into a single-cavity tablet press. The press can be activated at a pre-set pressure, e.g., 25 psi, and duration, e.g., 6 seconds, and the tablet formed and ejected from the press at room temperature. The ratio of vasodilator agent to PLGA can be 70/30 w/w for all compressed tablet implants. The tablet implant can be used as an intraocular implant to provide sustained release of the vasoactive agent at therapeutic levels to treat an ocular condition such as glaucoma.

### Example 2

### Manufacture of Extruded Implants

A vasoactive agent such as an endothelin receptor antagonists (eg Bosentan), a phosphodiester-5 inhibitor (eg Vardenafil) or pilocarpine and a poly(lactide-co-glycolide) (PLGA)can be weighed and placed in a stainless steel mixing vessel. The vessel can be sealed, placed on a Turbula mixer and mixed at a prescribed intensity, e.g., 96 rpm, and time, e.g., 10-15 minutes. The PLGA can comprise a 30/10 w/w mixture of hydrophilic end PLGA (Boehringer Ingelheim, Wallingford, CT) and hydrophobic end PLGA (Boehringer Ingelheim, Wallingford, CT). The resulting powder blend can be fed into a DACA Microcompounder-Extruder (DACA, Goleta, CA) and subjected to a pre-set temperature, e.g., 115°C, and screw speed, e.g., 12 rpm. The filament can be extruded into a guide mechanism and cut into exact lengths that corresponded to the designated implant weight. The ratio of vasoactive agent to total PLGA (hydrophilic and hydrophobic end) can be 60/40 w/w for all the extruded implants. The extruded implant can be used as an intraocular implant to provide sustained release of the vasoactive agent at therapeutic levels to treat an ocular condition such as glaucoma.

### Example 3

### Extrusion Process and Compression Method for Manufacturing Vasodilator-containing Biodegradable Intraocular Implants

Biodegradable implants are made by combining a vasodilator active agent with a biodegradable polymer composition in a stainless steel mortar. The combination is mixed via a Turbula shaker set at 96 RPM for 15 minutes. The powder blend is scraped off the wall of the mortar and then remixed for an additional 15 minutes. The mixed powder blend is heated to a semi-molten state at specified temperature for a total of 30 minutes, forming a polymer/drug melt.

Rods are manufactured by pelletizing the polymer/drug melt using a 9 gauge polytetrafluoroethylene (PTFE) tubing, loading the pellet into the barrel and extruding the material at the specified core extrusion temperature into filaments. The filaments are then cut into about 1 mg size implants or drug delivery systems. The rods have dimensions of about 2 mm long x 0.72 mm diameter. The rod implants weigh between about 500 µg and 1200 µg.

Wafers are formed by flattening the polymer melt with a Carver press at a specified temperature and cutting the flattened material into wafers, each weighing about 1 mg. The wafers have a diameter of about 2.5 mm and a thickness of about 0.13 mm. The wafer implants weigh between about 900 µg and 1100 µg.

In-vitro release testing can be performed on each lot of implant (rod or wafer). Each implant may be placed into a 24 mL screw cap vial with 10 mL of Phosphate Buffered Saline solution at 37°C and 1 mL aliquots are removed and replaced with equal volume of fresh medium on day 1, 4, 7, 14, 28, and every two weeks thereafter.

Drug assays can be performed by HPLC, which consists of a Waters 2690 Separation Module (or 2696), and a Waters 2996 Photodiode Array Detector. An Ultrasphere, C-18 (2), 4.6 x 150 mm column heated at 30 °C can be used for separation and the detector can be set at 264 nm. The mobile phase can be (10:90) MeOH - buffered mobile phase with a flow rate of 1 mL/min and a total run time of 12 min per sample. The buffered mobile phase may comprise (68:0.75:0.25:31) 13 mM 1-Heptane Sulfonic Acid, sodium salt - glacial acetic acid - triethylamine - Methanol. The release rates can be determined by calculating the amount of drug being released in a given volume of medium over time in µg/day.

The polymers chosen for the implants can be obtained from Boehringer Ingelheim or Purac America, for example. Examples of polymers include: RG502, RG752, R202H, R203 and R206, and Purac PDLG (50/50). RG502 is (50:50) poly(D,L-lactide-co-glycolide), RG752 is (75:25) poly(D,L-lactide-co-glycolide), R202H is 100% poly(D, L-lactide) with acid end group or terminal acid groups, R203 and R206 are both 100% poly(D, L-lactide). Purac PDLG (50/50) is (50:50) poly(D,L-lactide-co-glycolide). The inherent viscosity of RG502, RG752, R202H, R203, R206, and Purac PDLG are 0.2, 0.2, 0.2, 0.3, 1.0, and 0.2 dL/g, respectively. The average molecular weight of RG502, RG752, R202H, R203, R206, and Purac PDLG are, 11700, 11200, 6500, 14000, 63300, and 9700 daltons, respectively.

### Example 4

### Method for Placing Implants Into the Vitreous

Implants can be placed into the posterior segment of the right eye of New Zealand White Rabbits by incising the conjunctiva and sclera between the 10 and 12 o'clock positions with a 20-gauge microvitreoretinal (MVR) blade. Fifty to 100 µL of vitreous humor can be removed with a 1-cc syringe fitted with a 27-gauge needle. A sterile trocar, preloaded with and Example 1, 2 or 3 implant or implants, can be inserted 5 mm through the sclerotomy, and then retracted with the push wire in place, leaving the implant in the posterior segment. Sclerae and conjunctivae are than closed using a 7-0 Vicryl suture.

### Example 5

### Vasoactive PLA/PLGA intraocular Implants to Treat Glaucoma

A 72 year old female suffering from glaucoma in both eyes receives an intraocular implant containing a vasoactive agent and a combination of a PLA and PLGA in each eye. The implants weigh about 1 mg, and contain about 500 mg of a vasoactive agent such as Bosentan, Vardenafi or pilocarpine. One implant is placed in the vitreous of each eye using a syringe. In about two days, the patient reports a substantial relief in ocular comfort. Examination reveals that the intraocular pressure has decreased, the average intraocular pressure measured at 8:00 AM has decreased from 28 mm Hg to 14.3 mm Hg. The patient is monitored monthly for about 6 months. Intraocular pressure levels remain below 15 mm Hg for six months, and the patient reports reduced ocular discomfort.

### Example 6

### Vasodilator PLA Intraocular Implants for Increasing Retinal Blood Flow

A 62 year old male presents with peripheral field of vision loss in his left eye due to glaucoma. An implant containing 400 mg of pilocarpine and 600 mg of PLA is inserted into the vitreous of the left eye using the applicator shown in U.S. patent application 10/917,909 or 11/021,947. Laser Doppler Flowmetry (HRF) was used to measure blood flow in the fovea, superior and inferior retina regions. Thirty days after implantation retinal blood flow can increase by 10%-30% and the patient's vision does not deteriorate any further.

### Example 7

### Vasodilator PLGA Intraocular Implants for Improving Vision

A 69 year old male presents with peripheral field of vision loss due to glaucoma in both eyes. An implant containing 400 mg of pilocarpine and 600 mg of PLGA is inserted into the vitreous of each eye using the applicator shown in U.S. patent application 10/917,909 or 11/021,947. Laser Doppler Flowmetry (HRF) was used to measure blood flow in the fovea, superior and inferior retina regions. Thirty days after implantation retinal blood flow can increase by 10%-30% in each eye and over a period of six months the patient regains up to 50% of the lost visual field.

### Example 8

### Vasodilator PLGA Intraocular implants for Glaucoma Prophylaxis

A 51 year old male presents with glaucoma risk factors, including IOP of 22-24 mm Hg, family history of glaucoma, and observations of initial optic nerve cup damage, but no visual filed loss. The patient receives intravitreal implants in both eyes. The implants comprise 400 mg of pilocarpine and 600 mg of PLGA and are inserted into the vitreous of each eye using the applicator shown in U.S. patent application 10/917,909 or 11/021,947. Laser Doppler Flowmetry (HRF) was used to measure blood flow in the fovea, superior and inferior retina regions. Thirty days after implantation retinal blood flow can increase by 10%-30%. The patient is followed for two years during which time he shows no vision loss and no further optic nerve damage. Statistically this can be significant as patients with his risk factors can be expected to show some vision loss due to glaucoma.

### Example 9

### Retinal Genes Responsible for Decreased Retinal Blood Flow

We carried out an experiment which indicates that a vasoactive agent can be used to treat a retinal disorder, such as glaucoma by increasing retinal tissue blood flow. Thus we carried out a microarray study to compare gene expressions between normal and glaucoma retinas. Two normal control groups and four glaucoma groups were compared. Each of these six groups had pooled RNA from six patients. This study found that two families of genes were consistently changed in the glaucoma patient's group. Thus, all the hemoglobin family genes were reduced in the glaucoma groups whereas most of the metallothionein I family genes increased in the glaucoma groups. The data is presented in Tables 1 and 2.

Metallothioneins are zinc-binding peptides that are induced by ischemia and their artificial overexpression can prevent ischemia-induced damage. See eg Campagne M., et al., Evidence for a protective role of metallothionein-1 in focal cerebral ischemia, Proc Natl Acad Sci USA. 1999 Oct 26;96(22):12870-5; Carmel J., et al., Mediators of ischemic preconditioning identified by microarray analysis of rat spinal cord, Exp Neurol. 2004 Jan;185(1):81-96, and; Yanagitani S., et al., Ischemia induces metallothionein III expression in neurons of rat brain, Life Sci. 1999;64(8):707-15.

**Table 1 Expression levels of hemoglobin family genes on Affymetrix whole genome array**

| **Gene** | **Control** | **Control** | **Glaucoma** | **Glaucoma** | **Glaucoma** | **Glaucoma** |
|---|---|---|---|---|---|---|
| hemoglobin, beta | 11532 | 3648.3 | 1441.6 | 1551.8 | 2850.9 | 2426.1 |
| hemoglobin, beta | 6884.2 | 1529.1 | 755.3 | 731.8 | 1260.3 | 1117.1 |
| hemoglobin, alpha 1 | 8003.3 | 3558.1 | 1074.4 | 1217.8 | 1737.3 | 2330.0 |
| hemoglobin, beta | 9044.1 | 3009.2 | 1661.5 | 1424.1 | 2293.7 | 2494.8 |
| hemoglobin, alpha 2 | 6506.2 | 2539.9 | 1002.0 | 977.2 | 1746.6 | 2296.1 |
| hemoglobin, alpha 1 and hemoglobin, alpha 2 | 7440.7 | 2763.0 | 1152.5 | 1517.0 | 1803.0 | 1810.0 |
| hemoglobin, alpha 1 and hemoglobin, alpha 2 | 7353.6 | 3065.5 | 1112.3 | 1139.0 | 1920.1 | 2249.0 |

**Table 2 Expression levels of metallothionein family genes on Affymetrix whole genome array**

| **Name** | **Control** | **Control** | **Glaucoma** | **Glaucoma** | **Glaucoma** | **Glaucoma** |
|---|---|---|---|---|---|---|
| metallothionein 1X | 7834.9 | 6286.9 | 11380.7 | 9039.7 | 12427.1 | 11370.8 |
| metallothionein 1 G | 4512.0 | 4500.4 | 7725.8 | 6118.5 | 7998.1 | 5480.5 |
| metallothionein 1 H | 3448.0 | 3415.8 | 7005.1 | 4628.6 | 8965.6 | 5962.2 |
| metallothionein 1X | 7386.9 | 5689.0 | 11972.4 | 8590.2 | 13075.7 | 10892.7 |
| Homo sapiens metallothionein 1H-like protein mRNA | 5588.2 | 3879.3 | 8115.3 | 6127.6 | 9190.0 | 5618.2 |
| Homo sapiens metallothionein 2A (MT2A), mRNA. | 7892.4 | 7083.9 | 17922.3 | 11051.9 | 15454.9 | 13213.2 |
| metallothionein 1E (functional) | 5555.7 | 4443.6 | 9776.8 | 8517.9 | 8277.7 | 7289.8 |
| metallothionein 1F (functional) | 4312.8 | 3223.0 | 5103.6 | 6151.7 | 8225.5 | 4738.1 |
| Contains the gene for a novel protein with IBR domain, a gene for a novel protein similar to MT1E (metallothionein 1E (functional) | 2240.3 | 2347.2 | 4789.2 | 3379.0 | 4535.2 | 3271.3 |
| metallothionein 1F (functional) | 4222.4 | 3071.7 | 5880.2 | 4340.0 | 7415.8 | 3574.3 |
| metallothionein 1K | 689.0 | 395.8 | 1291.0 | 765.5 | 1299.3 | 1493.4 |

The Table 1 and Table 2 data were obtained using the following procedures and equipment. Eye samples from normal and glaucoma patients were obtained 1-6 hours after death and preserved in RNAlater reagent (Ambion, Inc., Austin, Texas). Next the retina of each eye sample was carefully dissected out and RNAs isolated Trizol reagent (Invitrogen Corporation, Carlsbad, California). The RNAs so obtained were treated with RNase-free DNase I in the presence of RNase inhibitors to remove contaminating DNAs The RNAs were then further purified by using RNAeasy kit (Qiagen, Inc., Valencia, California) and quantitated with Ribogreen kit (Molecular Probes, Inc., Eugene, Oregon).

Three pairs of RNA samples (from six eyes) were pooled to form a group and two control and four glaucoma groups were formed. The six groups of RNA samples were analyzed on Affymetrix HU133 2.0-Plus whole genome Genechips at Expression Analysis Inc, Durham, North Carolina. Bioinformatic analyses were also performed at Expression Analysis and differentially expressed genes between control and glaucoma samples were identified. The data in Tables 1 and 2 are measured fluorescent units showing genes that have altered expression levels in glaucoma samples comparing to the controls. The controls were obtained from patients not diagnosed with glaucoma. The fluorescent units were measured by a scanner.

The difference in the expression levels between the two Table 1 controls is a common variation as the controls are for pooled samples obtained from non-glaucoma patients.

The altered hemoglobin and metallothionein RNA levels in glaucoma patients indicate reduced blood flow influenced at transcriptional levels. Importantly, the glaucoma patients in this study were taking IOP-lowering medications and had normal IOP. Agents that increase blood flow had not been very successful in treating glaucoma mainly because there was no easy way to delivery vasoactive drugs to the back of the eye without affecting vascular systems in other tissues. By use of our intraocular implants disclosed herein we can specifically deliver vasoactive drugs to the back of the eye to increase local retinal blood flow in glaucoma and glaucoma risk patients.

Vasoactive agents could be any that increase blood flow, for example pilocarpine, endothelin receptor antagonists, phosphodiesterase-5 inhibitors. The amount the active agent (for several different, specific and suitable vasoactive agents) would need to be incorporated into an intraocular implant can be an amount which achieves a therapeutically effective intravitreal concentration. For an endothelin receptor antagonist such as Bosentan a 0.3-3 ug/ml concentration can be effective. See eg Giersbergen P., et al., Comparative investigation of the pharmacokinetics of Bosentan in Caucasian and Japanese healthy subjects, J Clin Pharmacol 2005; 45: 42-47). For a phosphodiesterase-5 inhibitor such as Vardenafil1-a 10 ug/L concentration can be effective. See eg Rajagopalan P., et al., Effect of high fat breakfast and moderate fat evening meal on the pharmacokinetics of vardenafil, an oral phosphodiesterase-5 inhibitor for the treatment of erectile dysfunction, J Clin Pharmacol 2003; 43: 260-267). For pilocarpine a 1-10 uM can be effective. See eg Yoshitomi T., et al., Pharmacological effects of pilocarpine on rabbit ciliary artery, Curr Eye Res 2000; 20(4): 254-259.

## Claims

1. An intraocular implant suitable for treating an ocular condition, the implant comprising:
(a) one or more vasodilators selected from a muscarinic agent, an endothelin receptor antagonist, a phosphodiester-5 inhibitor, a pharmacologically-active salt thereof and a pharmacologically-active ester thereof; and
(b) one or more biodegradable polymers selected from a polylactide (PLA), a polyglycolide (PGA) and a poly(lactide-co-glycolide) (PLGA), the biodegradable polymer(s) being associated with the vasodilator(s).

2. An implant according to claim 1, wherein after *in vivo* placement of the implant, the implant can release a therapeutically effective amount of the vasodilator for up to three years (±10%).

3. An implant according to claim 1, wherein the implant is structured to be placed in the vitreous of the eye.

4. An implant according to any preceding claim, wherein the muscarinic agent is pilocarpine.

5. An implant according to any preceding claim, wherein the endothelin receptor antagonist is sitaxsentan or bosentan.

6. An implant according to any preceding claim, wherein the phosphodiester-5 inhibitor is vardenafil, sildenafil or tadalafil.

7. A method for producing a biodegradable intravitreal implant, the method comprising the step of extruding a mixture of one or more vasodilator compounds and one or more biodegradable polymers, thereby forming an intravitreal implant suitable for treating an ocular condition;
wherein:
the vasodilator compound(s) is/are a muscarinic agent, an endothelin receptor antagonist, a phosphodiester-5 inhibitor, a pharmacologically-active salt thereof or a pharmacologically-active ester thereof;
the biodegradable polymer(s) is/are a polylactide (PLA), a polyglycolide (PGA) or a poly(lactide co-glycolide) (PLGA); and
after *in vivo* placement of the implant, the implant releases a therapeutically effective amount of the vasodilator compound(s) over a period of up to forty days (±10%).

8. A method according to claim 7, wherein the biodegradable polymer(s) is/are a polylactide or a poly(lactide co-glycolide).

9. A biodegradable intraocular implant as defined in claim 1 for use in a method of improving or maintaining the vision of a patient, wherein the method comprises the step of placing the implant in an eye of the patient, and the implant degrades at a rate effective to sustain the release of an amount of the vasodilator from the implant so as to improve or maintain vision.

10. A biodegradable implant for use according to claim 9, wherein the method is effective to treat a retinal ocular condition.

11. A biodegradable implant for use according to claim 10, wherein the ocular condition includes retinal damage.

12. A biodegradable intraocular implant as defined in claim 1 for use in a method of treating glaucoma, the method comprising intravitreal administration of the implant.

13. A biodegradable intraocular implant as defined in claim 1 for use in a method of preventing vision loss, the method comprising intraocular placement of the implant.

## Patentansprüche

1. Intraokularimplantat, das geeignet ist einen Augenzustand zu behandeln, wobei das Implantat folgendes umfasst:
(a) einen oder mehrere Vasodilatoren, die aus einem Muskarinikum, einem Endothelinrezeptor-Antagonisten, einem Phosphodiester-5-Inhibitor, einem pharmakologisch aktiven Salz davon und einem pharmakologisch aktiven Ester davon ausgewählt sind; und
(b) ein oder mehrere biologisch abbaubare Polymere, die aus einem Polylactid (PLA), einem Polygylcolid (PGA) und einem Poly(lactid-co-glycolid) (PLGA) ausgewählt sind, wobei das/die biologisch abbaubaren Polymer(e) mit dem(den) Vasodilator(en) verbunden sind.

2. Implantat gemäß Anspruch 1, wobei nach dem *in vivo-*Einbringen des Implantats, das Implantat eine therapeutisch wirksame Menge des Vasodilators über bis zu drei Jahre (±10%) abgeben kann.

3. Implantat gemäß Anspruch 1, wobei das Implantat so aufgebaut ist, dass es in den Glaskörper des Auges eingebracht werden kann.

4. Implantat gemäß einem der vorhergehenden Ansprüche, wobei das Muskarinikum Pilocarpin ist.

5. Implantat gemäß einem der vorhergehenden Ansprüche, wobei der Endothelinrezeptor-Antagonist Sitaxsentan oder Bosentan ist.

6. Implantat gemäß einem der vorhergehenden Ansprüche, wobei der Phosphodiester-5-Inhibitor Vardenafil, Slidenafil oder Tadalafil ist.

7. Verfahren zur Herstellung eines biologisch abbaubaren intravitrealen Implantats, wobei das Verfahren den Schritt des Extrudierens einer Mischung einer oder mehrerer Vasodilatorverbindungen und eines oder mehrerer biologisch abbaubarer Polymere umfasst, um dadurch ein intravitreales Implantat zu bilden, das zur Behandlung eines Augenzustands geeignet ist;
wobei:
die Vasodilatorverbindung(en) ein Muskarinikum, ein Endothelinrezeptor-Antagonist, ein Phosphodiester-5-Inhibitor, ein pharmakologisch aktives Salz davon oder ein pharmakologisch aktiver Ester davon ist/sind;
das/die biologisch abbaubare(n) Polymer(e) ein Polylactid (PLA), ein Polyglycolid (PGA) oder ein Poly(lactid-co-glycolid) (PLGA) ist/sind; und
nach dem in vivo-Einbringen des Implantats, das Implantat eine therapeutisch wirksame Menge der Vasodilatorverbindung(en) über einen Zeitraum von bis zu vierzig Tagen (±10%) abgibt.

8. Verfahren gemäß Anspruch 7, wobei das/die biologisch abbaubare(n) Polymer(e) ein Polylactid oder ein Poly(lactid-co-glycolid) ist/sind.

9. Biologisch abbaubares Intraokularimplantat gemäß Anspruch 1 zur Verwendung in einem Verfahren zur Verbesserung oder Erhaltung des Sehvermögens eines Patienten, wobei das Verfahren den Schritt des Einbringens des Implantats in das Auge des Patienten umfasst, und das Implantat sich mit einer Geschwindigkeit zersetzt, die effektiv ist, um die Abgabe einer Menge des Vasodilators aus dem Implantat so aufrecht zu erhalten, dass das Sehvermögen verbessert oder erhalten wird.

10. Biologisch abbaubares Implantat zur Verwendung gemäß Anspruch 9, wobei das Verfahren für die Behandlung eines Augenzustandes der Netzhaut wirksam ist.

11. Biologisch abbaubares Implantat zur Verwendung gemäß Anspruch 10, wobei der Augenzustand Schäden der Netzhaut einschließt.

12. Biologisch abbaubares Intraokularimplantat gemäß Anspruch 1 zur Verwendung in einem Verfahren zur Behandlung eines Glaukoms, wobei das Verfahren die intravitreale Anwendung des Implantats umfasst.

13. Biologisch abbaubares Intraokularimplantat gemäß Anspruch 1 zur Verwendung in einem Verfahren zur Verhinderung des Verlustes des Sehvermögens, wobei das Verfahren das intraokulare Einbringen des Implantats umfasst.

## Revendications

1. Implant intraoculaire permettant de traiter un trouble oculaire, l'implant comprenant :
(a) un ou plusieurs vasodilatateurs choisis parmi un agent muscarinique, un antagoniste du récepteur de l'endothéline, un inhibiteur du phosphodiester-5, un sel pharmacologiquement actif de ceux-ci et un ester pharmacologiquement actif de ceux-ci ; et
(b) un ou plusieurs polymères biodégradables choisis parmi un polylactide (PLA), un polyglycolide (PGA) et un poly(lactide-co-glycolide) (PLGA), le(s) polymère(s) biodégradable(s) étant associé(s) au(x) vasodilatateur(s).

2. Implant selon la revendication 1, dans lequel, après le placement *in vivo* de l'implant, l'implant peut libérer une quantité thérapeutiquement efficace du vasodilatateur pouvant aller jusqu'à trois ans (± 10 %).

3. Implant selon la revendication 1, dans lequel l'implant est structuré pour être placé dans le corps vitré de l'oeil.

4. Implant selon l'une quelconque des revendications précédentes, dans lequel l'agent muscarinique est la pilocarpine.

5. Implant selon l'une quelconque des revendications précédentes, dans lequel l'antagoniste du récepteur de l'endothéline est le sitaxsentan ou le bosentan.

6. Implant selon l'une quelconque des revendications précédentes, dans lequel l'inhibiteur de phosphodiester-5 est le vardénafil, le sildénafil ou le tadalafil.

7. Procédé de fabrication d'un implant intravitréen biodégradable, le procédé comprenant l'étape d'extrusion d'un mélange d'un ou plusieurs composés vasodilatateurs et d'un ou plusieurs polymères biodégradables, en formant ainsi un implant intravitréen permettant de traiter un trouble oculaire;
dans lequel :
le(s) composé(s) vasodilatateur(s) est/sont un agent muscarinique, un antagoniste du récepteur de l'endothéline, un inhibiteur du phosphodiester-5, un sel pharmacologiquement actif de ceux-ci ou un ester pharmacologiquement actif de ceux-ci ;
le(s) polymère(s) biodégradable(s) est/sont un polylactide (PLA), un polyglycolide (PGA) ou un poly (lactide co-glycolide) (PLGA); et
après le placement *in vivo* de l'implant, l'implant libère une quantité thérapeutiquement efficace du (des) composé(s) vasodilatateur(s) sur une période allant jusqu'à quarante jours (± 10 %).

8. Procédé selon la revendication 7, dans lequel le(s) polymère(s) biodégradable(s) est/sont un polylactide ou un poly(lactide co-glycolide).

9. Implant intraoculaire biodégradable tel que défini dans la revendication 1 pour une utilisation dans un procédé d'amélioration ou de maintien de la vision d'un patient, dans lequel le procédé comprend l'étape comprenant le placement de l'implant dans un oeil du patient, et l'implant se dégrade à une vitesse suffisante pour permettre la libération d'une quantité de vasodilatateur à partir de l'implant afin d'améliorer ou de maintenir la vision.

10. Implant biodégradable à utiliser selon la revendication 9, dans lequel le procédé est efficace pour traiter un trouble oculaire de la rétine.

11. Implant biodégradable à utiliser selon la revendication 10, dans lequel le trouble oculaire comprend un endommagement de la rétine.

12. Implant intraoculaire biodégradable tel que défini dans la revendication 1 pour une utilisation dans un procédé de traitement du glaucome, le procédé comprenant une administration intravitréenne de l'implant.

13. Implant intraoculaire biodégradable tel que défini dans la revendication 1, pour une utilisation dans un procédé de prévention de la perte de vision, le procédé comprenant le placement intraoculaire de l'implant.
